# FASCICULE DE BREVET EUROPEEN

(11) **EP 1 047 384 B1**
(45) Date de publication et mention de la délivrance du brevet: **25.02.2004**
(21) Numéro de dépôt: 98963599.0
(22) Date de dépôt: 22.12.1998
(51) Int. Cl.: A61K 7/13

(54) **COMPOSITION DE TEINTURE D'OXYDATION DES FIBRES KERATINIQUES CONTENANT UNE LACCASE ET PROCEDE DE TEINTURE METTANT EN OEUVRE CETTE COMPOSITION**
OXIDATIONSFÄRBEMITTEL FÜR KERATINFASERN, DAS EINE LACCASE ENTHÄLT, UND VERFAHREN ZUR FÄRBUNG MIT DIESEM MITTEL
KERATINOUS FIBRE OXIDATION DYEING COMPOSITION CONTAINING A LACCASE AND DYEING METHOD USING SAME

(30) Priorité: 13.01.1998 FR 9800256
(43) Date de publication de la demande: 02.11.2000
(73) Titulaire: L'OREAL, 75008 Paris (FR)
(72) Inventeur: LANG, Gérard, F-95390 Saint Prix (FR); COTTERET, Jean, F-78480 Verneuil sur Seine (FR)
(74) Mandataire: Miszputen, Laurent
(86) Numéro de dépôt international: PCT/FR1998/002834
(87) Numéro de publication internationale: WO 1999/036042

(56) Documents cités:
- EP-A- 0 504 005
- EP-A- 0 795 313
- DE-C- 19 647 494
- FR-A- 2 694 018

## Description

L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation, du 2-amino 4-N-(β-hydroxyéthyl)amino anisole à titre de coupleur, et au moins une enzyme de type laccase, ainsi que le procédé de teinture mettant en oeuvre cette composition.

Il est connu de teindre les fibres kératiniques et en particulier les cheveux humains avec des compositions tinctoriales contenant des précurseurs pour la coloration d'oxydation, en particulier des ortho ou paraphénylènediamines, des ortho ou paraaminophénols, des bases hétérocycliques, appelés généralement bases d'oxydation. Les précurseurs pour la coloration d'oxydation, (bases d'oxydation), sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé dans le brevet US 3251742, les demandes de brevet FR-A-2 112 549, FR-A-2 694 018, EP-A-0 504 005, WO95/07988, WO95/33836, WO95/33837, WO96/00290, WO97/19998 et WO97/19999 de teindre les fibres kératiniques avec des compositions comprenant au moins un colorant d'oxydation, ou au moins un précurseur de mélanine, en association avec des enzymes du type laccase ; lesdites compositions étant mises en contact avec l'oxygène de l'air. Ces formulations de teinture, bien qu'étant mises en oeuvre dans des conditions n'entraînant pas une dégradation des fibres kératiniques comparable à celle engendrée par les teintures réalisées en présence de peroxyde d'hydrogène, conduisent à des colorations encore insuffisantes à la fois sur le plan de l'homogénéité de la couleur répartie le long de la fibre (unisson), sur le plan de la chromaticité (luminosité) et de la puissance tinctoriale.

Or, la demanderesse vient maintenant de découvrir qu'il est possible d'obtenir de nouvelles teintures, capables de conduire à des colorations puissantes sans engendrer de dégradation significative des fibres kératiniques, peu sélectives et résistant bien aux diverses agressions que peuvent subir les fibres, en associant au moins une base d'oxydation, du 2-amino 4-N-(β-hydroxyéthyl)amino anisole à titre de coupleur, et au moins une enzyme de type laccase.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation,
- du 2-amino 4-N-(β-hydroxyéthyl)amino anisole et/ou au moins l'un de ses sels d'addition avec un acide à titre de coupleur,
- au moins une enzyme de type laccase.

La composition tinctoriale prête à l'emploi conforme à l'invention conduit à des colorations puissantes présentant une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition tinctoriale prête à l'emploi.

La ou les laccases utilisées dans la composition tinctoriale prête à l'emploi conforme à l'invention peuvent notamment être choisies parmi les laccases d'origine végétale, d'origine animale, d'origine fongique (levures, moisissures, champignons) ou d'origine bactérienne, les organismes d'origine pouvant être mono- ou pluricellulaires. La ou les laccases utilisées dans la composition tinctoriale prête à l'emploi conforme à l'invention peuvent également être obtenues par biotechnologie.

Parmi les laccases d'origine végétale utilisables selon l'invention, on peut citer les laccases produites par des végétaux effectuant la synthèse chlorophyllienne telles que celles indiquées dans la demande de brevet FR-A-2 694 018.

On peut notamment citer les laccases présentes dans les extraits d'Anacardiacées tels que par exemple les extraits de Magnifera indica, de Schinus molle ou de Pleiogynium timoriense ; dans les extraits de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

Parmi les laccases d'origine fongique, éventuellement obtenues par biotechnologie, utilisables selon l'invention, on peut citer la ou les laccases issues de Polyporus versicolor, de Rhizoctonia praticola et de Rhus vernicifera telles que décrites par exemples dans les demandes de brevet FR-A-2 112 549 et EP-A-504005 ; les laccases décrites dans les demandes de brevet WO95/07988, WO95/33836, WO95/33837, WO96/00290, WO97/19998 et WO97/19999, dont le contenu fait partie intégrante de la présente description comme par exemple la ou les laccases issues de Scytalidium, de Polyporus pinsitus, de Myceliophtora thermophila, de Rhizoctonia solani, de Pyricularia orizae, et leurs variantes. On peut également citer la ou les laccases issues de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

On choisira plus préférentiellement les laccases d'origine fongiques, éventuellement obtenues par biotechnologie.

L'activité enzymatique des laccases utilisées conformément à l'invention et ayant la syringaldazine parmi leurs substrat peut être définie à partir de l'oxydation de la syringaldazine en condition aérobie. L'unité Lacu correspond à la quantité d'enzyme catalysant la conversion de 1 mmole de syringaldazine par minute à un pH de 5,5 et à une température de 30°C. L'unité U correspond à la quantité d'enzyme produisant un delta d'absorbance de 0,001 par minute, à une longueur d'onde de 530 nm, en utilisant la syringaldazine comme substrat, à 30°C et à un pH de 6,5. L'activité enzymatique des laccases de l'invention peut aussi être définie à partir de l'oxydation de la paraphénylènediamine. L'unité ulac correspond à la quantité d'enzyme produisant un delta d'absorbance de 0,001 par minute, à une longueur d'onde de 496,5 nm, en utilisant la paraphénylènediamine comme substrat (64 mM), à 30°C et à un pH de 5.

Selon l'invention, on préfère déterminer l'activité enzymatique en unités ulac.

La quantité de laccase(s) présente dans la composition tinctoriale prête à l'emploi conforme à l'invention variera en fonction de la nature de la ou des laccases utilisées. De façon préférentielle, la quantité de laccase(s) est comprise entre 0,5 et 200 Lacu environ (soit entre 10000 et 4.10⁶ unités U environ ou soit entre 20 et 2.10⁶ unités ulac) pour 100 g de composition tinctoriale.

La nature de la ou des bases d'oxydation utilisées dans la composition tinctoriale prête à l'emploi n'est pas critique. Elles peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (1) suivante et leurs sels d'addition avec un acide : 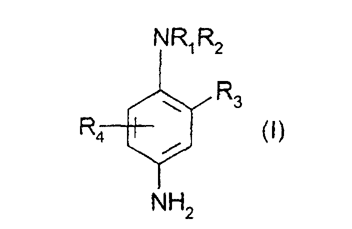 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 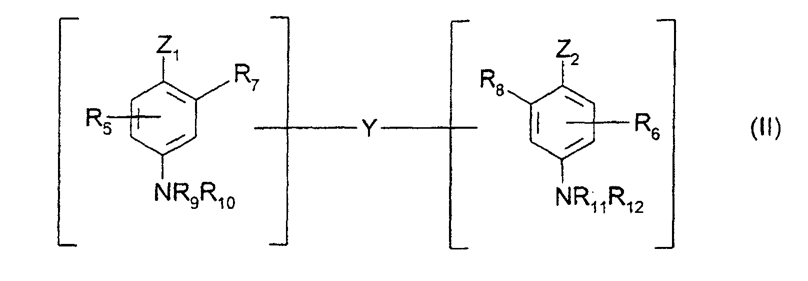 dans laquelle :
- Z₁ et Z₂, identiques ou différents, représentent un radical hydroxyle ou -NH₂ pouvant être substitué par un radical alkyle en C₁-C₄ ou par un bras de liaison Y;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N, N'-bis-( éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.
Parmi les para-aminophénols utilisables à titre de bases d'oxydation dans les compositions tinctoriales conformes à l'invention, on peut notamment citer les composés répondant à la formule (III) suivante, et leurs sels d'addition avec un acide : 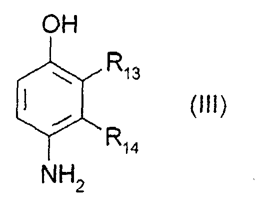 dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide. Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement mentionner les composés décrits dans la demande de brevet FR-A-2 750 048, parmi lesquels on peut citer :
- la pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1 ,5-a]pyrimidin-7 -yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;
et leurs sels d'addition et leurs formes tautomères, lorsqu'il existe un équilibre tautomérique.

La ou les bases d'oxydation représentent de préférence de 0,0005 à 12 % en poids environ du poids total de la composition tinctoriale conforme à l'invention, et encore plus préférentiellement de 0,005 à 6 % en poids environ de ce poids.

Le 2-amino 4-N-(β-hydroxyéthyl)amino anisole et/ou le ou ses sels d'addition avec un acide, représentent de préférence de 0,0001 à 8 % en poids environ du poids total de la composition tinctoriale, et encore plus préférentiellement de 0,005 à 5 % en poids environ de ce poids.

La composition tinctoriale conforme à l'invention peut contenir un ou plusieurs autres coupleurs différents du 2-amino 4-N-(β-hydroxyéthyl)amino anisole et/ou des colorants directs notamment pour modifier les nuances ou les enrichir en reflets.

Parmi les coupleurs pouvant être présents à titre additionnel dans la composition tinctoriale prête à l'emploi conforme à l'invention, on peut notamment citer les méta-phénylènediamines, les méta-aminophénols, les métadiphénols, et leurs sels d'addition avec un acide.

Lorsqu'ils sont présents ces coupleurs additionnels représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau.

Le pH de la composition prête à l'emploi conforme à l'invention est choisi de telle manière que l'activité enzymatique de la laccase soit suffisante, Il est généralement compris entre 4 et 11 environ, et de préférence entre 6 et 9 environ. Il peut être ajusté à la valeur désirée au moyen d'agents acidifiants ou alcalinisants habituellement utilisés en teinture des fibres kératiniques.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères, des agents antioxydants, des enzymes différentes des laccases utilisées conformément à l'invention telles que par exemples des peroxydases ou des oxydo-réductases à 2 électrons, des agents de pénétration, des agents séquestrants, des parfums, des tampons, des agents dispersants, des agents épaississants, des agents filmogènes, des agents conservateurs, des agents opacifiants, des vitamines.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, les colorants d'oxydation et la ou les enzymes de type laccase sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins une base d'oxydation et du 2-amino 4-N-(β-hydroxyéthyl)amino anisole et/ou au moins un de ses sels d'addition avec un acide, d'autre part, une composition (B) renfermant, dans un milieu approprié pour la teinture, au moins une enzyme de type laccase, puis à procéder à leur mélange au moment de l'emploi avant d'appliquer ce mélange sur les fibres kératiniques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

L'exemple qui suit est destiné à illustrer l'invention sans pour autant en limiter la portée.

### EXEMPLE DE TEINTURE

On a préparé la composition tinctoriale suivante :
- Paraphénylènediamine 0,2 g
- Dichlorhydrate de 2-amino 4-N-(β-hydroxyéthyl)amino anisole 0,25 g
- Laccase issue de Rhus vernicifera à 180 unités / mg vendue par la société Sigma 1,8 g
- Alkyl (C₈-C₁₀) polyglucoside en solution aqueuse à 60 % de matière active (M.A.), vendu sous la dénomination ORAMIX CG110® par la société SEPPIC 8,0 g
- Ethanol 20 g
- Agent de pH q.s. pH 6,5
- Eau déminéralisée q.s.p. 100 g

La composition tinctoriale prêtes à l'emploi décrite ci-dessus a été appliquée sur des mèches de cheveux gris naturels à 90 % de blancs pendant 40 minutes, à une température de 30°C. Les cheveux ont ensuite été rincés, puis séchés.

Les cheveux ont été teints dans une nuance gris bleu.

Dans la composition tinctoriale décrite ci-dessus, la laccase de Rhus vernicifera à 180 unités / mg, vendue par la société Sigma peut être remplacée par 1,0 g de laccase de Pyricutaria orizae à 100 unités / mg vendue par la société ICN.

## Revendications

1. Composition prête à l'emploi, pour la teinture d'oxydation des fibres kératiniques et en particulier des fibres kératiniques humaines telles que les cheveux, **caractérisée par le fait qu'**elle comprend, dans un milieu approprié pour la teinture :
- au moins une base d'oxydation,
- du 2-amino 4-N-(β-hydroxyéthyl)amino anisole et/ou au moins l'un de ses sels d'addition avec un acide à titre de coupleur,
- au moins une enzyme de type laccase.

2. Composition selon la revendication 1, **caractérisée par le fait que** la laccase est choisie parmi les laccases d'origine végétale, d'origine animale, d'origine fongique ou d'origine bactérienne et parmi les laccases obtenues par biotechnologie.

3. Composition selon l'une quelconque des revendications 1 ou 2, **caractérisée par le fait que** la laccase est d'origine végétale et choisie parmi les laccases présentes dans les extraits d'Anacardiacées ; de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

4. Composition selon la revendication 1 ou 2, **caractérisée par le fait que** la laccase est d'origine fongique ou obtenue par biotechnologie.

5. Composition selon la revendication 4, **caractérisée par le fait que** la laccase est choisie parmi les laccases issues de Polyporus versicolor, de Rhizoctonia praticola, de Rhus vernicifera, de Scytalidium, de Polyporus pinsitus, de Myceliophtora thermophila, de Rhizoctonia solani, de Pyricularia orizae, de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

6. Composition selon l'une quelconque des revendications précédentes, **caractérisée par le fait que** la quantité de laccase(s) est comprise entre 0,5 et 200 Lacu pour 100 g de composition tinctoriale.

7. Composition selon l'une quelconque des revendications précédentes, **caractérisée par le fait que** la ou les bases d'oxydation sont choisies parmi les paraphénylènediamines, les bases doubles, les para-aminophénols, les ortho aminophénols et les bases d'oxydation hétérocycliques.

8. Composition selon la revendication 7, **caractérisée par le fait que** les paraphénylènediamines sont choisies parmi les composés de formule (I), et leurs sels d'addition avec une acide : 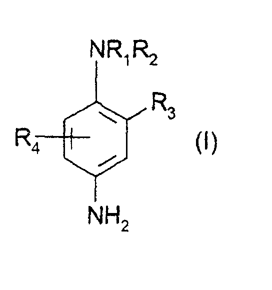 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

9. Composition selon la revendication 8, **caractérisée par le fait que** les paraphénylènediamines de formule (I) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

10. Composition selon la revendication 7, **caractérisée par le fait que** les bases doubles sont choisies parmi les composés de formule (II), et leurs sels d'addition avec un acide : 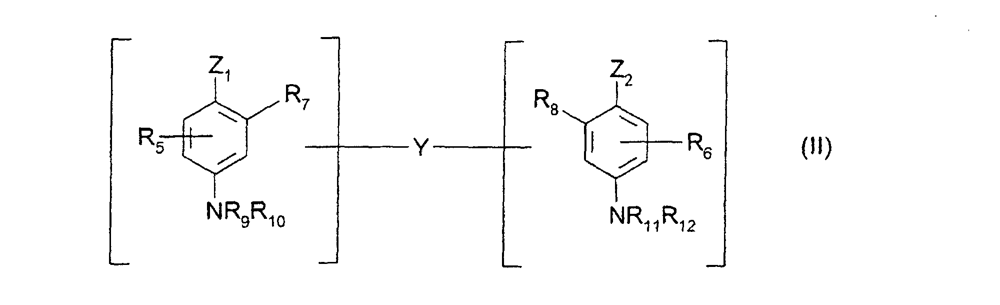 dans laquelle :
- Z₁ et Z₂, identiques ou différents, représentent un radical hydroxyle ou -NH₂ pouvant être substitué par un radical alkyle en C₁-C₄ au par un bras de liaison Y;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ; étant entendu que les composés de formule (II) ne comportent qu'un seul bras de liaison Y par molécule.

11. Composition selon la revendication 10, **caractérisée par le fait que** les bases doubles de formule (II) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

12. Composition selon la revendication 7, **caractérisée par le fait que** les para-aminophénols sont choisis parmi les composés de formule (III), et leurs sels d'addition avec un acide : 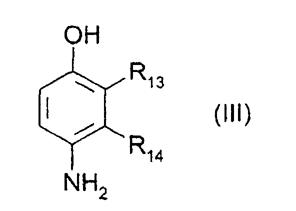 dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄ ;
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄) ; étant entendu qu'au moins un des radicaux R₁₃ ou R₁₄ représente un atome d'hydrogène.

13. Composition selon la revendication 12, **caractérisée par le fait que** les para-aminophénols de formule (III) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

14. Composition selon la revendication 7, **caractérisée par le fait que** les orthoaminophénols sont choisis parmi 2-amino phénol, le 2-amino 5-méthyl phénol, le 2-amino 6-méthyl phénol, le 5-acétamido 2-amino phénol, et leurs sels d'addition avec un acide.

15. Composition selon la revendication 7, **caractérisée par le fait que** les bases hétérocycliques sont choisies parmi les dérivés pyridiniques, les dérivés pyrimidiniques, les dérivés pyrazoliques, les dérivés pyrazolo-pyrimidiniques, et leurs sels d'addition avec un acide.

16. Composition selon l'une quelconque des revendications précédentes, **caractérisée par le fait que** la ou les bases d'oxydation représentent de 0,0005 à 12 % en poids du poids total de la composition tinctoriale.

17. Composition selon la revendication 16, **caractérisée par le fait que** la ou les bases d'oxydation représentent de 0,005 à 6 % en poids du poids total de la composition tinctoriale.

18. Composition selon l'une quelconque des revendications précédentes, **caractérisée par le fait que** le 2-amino 4-N-(β-hydroxyéthyl)amino anisole et/ou le ou ses sels d'addition avec un acide, représentent de 0,0001 à 8 % en poids du poids total de la composition tinctoriale.

19. Composition selon la revendication 18, **caractérisée par le fait que** le 2-amino 4-N-(β-hydroxyéthyl)amino anisole et/ou le ou ses sels d'addition avec un acide, représentent de 0,005 à 5 % en poids du poids total de la composition tinctoriale.

20. Composition selon l'une quelconque des revendications précédentes, **caractérisée par le fait qu'**elle contient un ou plusieurs autres coupleurs différents du 2-amino 4-N-(β-hydroxyéthyl)amino anisole et/ou des colorants directs.

21. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

22. Composition selon l'une quelconque des revendications précédentes, **caractérisée par le fait que** le milieu approprié pour la teinture est constitué par de l'eau ou par un mélange d'eau et d'au moins un solvant organique.

23. Composition selon l'une quelconque des revendications précédentes, **caractérisée par le fait qu'**elle présente un pH compris 4 et 11.

24. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications précédentes, pendant un temps suffisant pour développer la coloration désirée.

25. Procédé selon la revendication 24, **caractérisé par le fait qu'**il comporte une étape préliminaire consistant à stocker sous forme séparée, d'une part, une composition (A) comprenant, dans un milieu approprié pour la teinture au moins une base d'oxydation et du 2-amino 4-N-(β-hydroxyéthyl)amino anisole et/ou au moins un de ses sels d'addition avec un acide et, d'autre part, une composition (B) renfermant, dans un milieu approprié pour la teinture, au moins une enzyme de laccase, puis à procéder à leur mélange au moment de l'emploi avant d'appliquer ce mélange sur les fibres kératiniques.

26. Dispositif à plusieurs compartiments ou "kit" de teinture, **caractérisé par le fait qu'**il comporte un premier compartiment renfermant la composition (A) telle que définie dans la revendication 25 et un second compartiment renfermant la composition (B) telle que définie dans la revendication 25.

## Patentansprüche

1. Gebrauchsfertige Zusammensetzung zum oxidativen Färben von Keratinfasern und insbesondere menschlichen Keratinfasern, wie dem Haar, **dadurch gekennzeichnet, dass** sie in einem zum Färben geeigneten Medium enthält:
- mindestens eine Oxidationsbase,
- das 2-Amino-4-N-(β-hydroxyethyl)amino-anisol und/oder mindestens ein Additionssalz dieser Verbindung mit einer Säure als Kuppler, und
- mindestens ein Enzym vom Laccase- Typ.

2. Zusammensetzung nach Anspruch 1, **dadurch gekennzeichnet, dass** die Laccase unter den Laccasen pflanzlicher Herkunft, den Laccasen tierischer Herkunft, den Laccasen, die von Pilzen gebildet werden, den Laccasen bakterieller Herkunft oder den biotechnologisch hergestellten Laccasen ausgewählt ist.

3. Zusammensetzung nach einem der Ansprüche 1 oder 2, **dadurch gekennzeichnet, dass** die Laccase pflanzlicher Herkunft ist und unter den Laccasen ausgewählt ist, die in Extrakten von Anacardiaceae, Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica und Pistacia palaestina vorliegen.

4. Zusammensetzung nach Anspruch 1 oder 2, **dadurch gekennzeichnet, dass** die Laccase von Pilzen gebildet oder biotechnologisch hergestellt ist.

5. Zusammensetzung nach Anspruch 4, **dadurch gekennzeichnet, dass** die Laccase unter den Laccasen ausgewählt ist, die von Polyporus versicolor, Rhizoctonia praticola, Rhus vernicifera, Scytalidium, Polyporus pinsitus, Myceliophtora thermophila, Rhizoctonia solani, Pyricularia orizae, Trametes versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitus squalens und deren Varianten stammen.

6. Zusammensetzung nach einem der vorhergehenden Ansprüche, **dadurch gekennzeichnet, dass** die Laccase(n) in einem Mengenanteil im Bereich von 0,5 bis 200 lacu pro 100 g Farbmittelzusammensetzung vorliegen.

7. Zusammensetzung nach einem der vorhergehenden Ansprüche, **dadurch gekennzeichnet, dass** die Oxidationsbase(n) unter den p-Phenylendiaminen, Doppelbasen, p-Aminophenolen, o-Aminophenolen und den heterocyclischen Oxidationsbasen ausgewählt sind.

8. Zusammensetzung nach Anspruch 7, **dadurch gekennzeichnet, dass** die p-Phenylendiamine unter den Verbindungen der folgenden Formel (I) und deren Additionssalzen mit einer Säure ausgewählt sind: 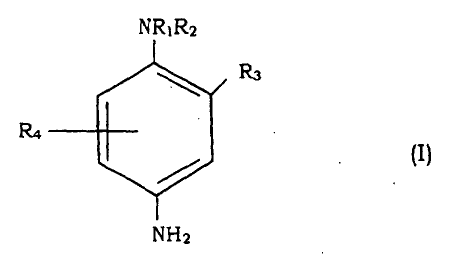 worin bedeuten:
- R₁ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl, eine mit einer stickstoffhaltigen Gruppe substituierte C₁₋₄-Alkylgruppe, Phenyl oder 4'-Aminophenyl,
- R₂ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl oder eine mit einer stickstoffhaltigen Gruppe substituierte C₁₋₄-Alkylgruppe,
- R₃ Wasserstoff, Halogen, wie Chlor, Brom, Iod oder Fluor, C₁₋₄-Alkyl, C₁₋₄-Monohydroxyalkyl, C₁₋₄-Hydroxyalkoxy, C₁₋₄-Acetylaminoalkoxy, C₁₋₄-Mesylaminoalkoxy oder C₁₋₄-Carbamoylaminoalkoxy, und
- R₄ Wasserstoff, Halogen oder C₁₋₄-Alkyl.

9. Zusammensetzung nach Anspruch 8, **dadurch gekennzeichnet, dass** die p-Phenylendiamine der Formel (I) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-pphenylendiamin, 2,5-Dimethyl-p-phenylendiamin, N,N-Dimethyl-pphenylendiamin, N,N-Diethyl-p-phenylendiamin, N,N-Dipropyl-pphenylendiamin, 4-Amino-N,N-diethyl-3-methyl-anilin, N,N-Bis(β-hydroxyethyl)-p-phenylendiamin, 4-N,N-bis(β-Hydroxyethyl)amino-2-methyl-anilin, 4-N,N-bis(β-Hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-pphenylendiamin, N-(β-Methoxyethyl)-p-phenylendiamin und deren Additionssalzen mit einer Säure ausgewählt sind.

10. Zusammensetzung nach Anspruch 7, **dadurch gekennzeichnet, dass** die Doppelbasen unter den Verbindungen der folgenden Formel (II) und deren Additionssalze mit einer Säure ausgewählt sind: 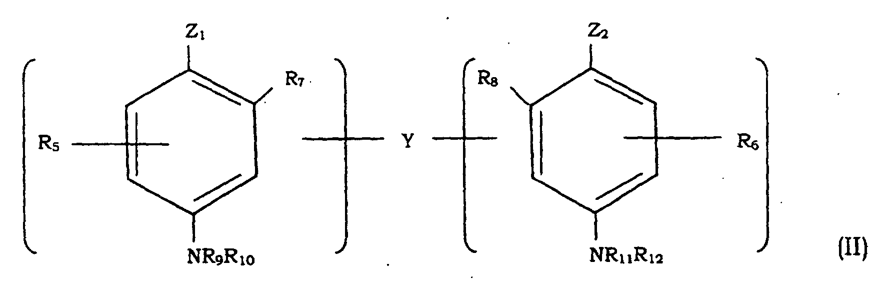 worin bedeuten:
- Z₁ und Z₂, die identisch oder voneinander verschieden sind, Hydroxy oder eine NH₂-Gruppe, die mit C₁₋₄-Alkyl oder einer Verbindungsgruppe Y substituiert sein kann,
- die Verbindungsgruppe Y eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden kann und gegebenenfalls mit einer oder mehreren Hydroxy- oder C₁₋₆-Alkoxygruppen substituiert sein kann,
- R₅ und R₆ Wasserstoff, Halogen, C₁₋₄-Alkyl, C₁₋₄-Monohydroxyalkyl, C₂₋₄-Polyhydroxyalkyl, C₁₋₄-Aminoalkyl oder eine Verbindungsgruppe Y,
- R₇, R₈, R₉, R₁₀, R₁₁ und R₁₂, die identisch oder voneinander verschieden sind, Wasserstoff, eine Verbindungsgruppe Y oder C₁₋₄-Alkyl,
- mit der Maßgabe, dass die Verbindungen der Formel (II) nur eine Verbindungsgruppe Y pro Molekül aufweisen.

11. Zusammensetzung nach Anspruch 10, **dadurch gekennzeichnet, dass** die Doppelbasen der Formel (II) unter N,N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis-(4'amino, 3'-methylphenyl)-ethylendiamin, 1,8-Bis(2,5-diaminophenoxy)-3,5-dioxaoctan und deren Additionssalzen mit einer Säure ausgewählt sind.

12. Zusammensetzung nach Anspruch 7, **dadurch gekennzeichnet, dass** die p-Aminophenole unter den Verbindungen der folgenden Formel (III) und deren Additionssalze mit einer Säure ausgewählt sind: 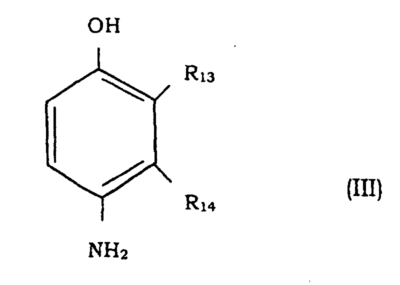 worin bedeuten:
- R₁₃ Wasserstoff, Halogen, C₁₋₄-Alkyl, C₁₋₄-Monohydroxyalkyl, C₁₋₄-Alkoxy-C₁₋₄-alkyl, C₁₋₄-Aminoalkyl oder C₁₋₄-Hydroxyalkyl-C₁₋₄aminoalkyl,
- R₁₄ Wasserstoff, Halogen, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder C₁₋₄-Alkoxy-C₁₋₄-alkyl,
- mit der Maßgabe, dass mindestens eine der Gruppen R₁₃ oder R₁₄ Wasserstoff bedeutet.

13. Zusammensetzung nach Anspruch 12, **dadurch gekennzeichnet, dass** die p-Aminophenole der Formel (III) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalzen mit einer Säure ausgewählt sind.

14. Zusammensetzung nach Anspruch 7, **dadurch gekennzeichnet, dass** die o-Aminophenole unter 2-Aminophenol, 2-Amino-5-methylphenol, 2-Amino-6-methyl-phenol, 5-Acetamido-2-amino-phenol und deren Additionssalzen mit einer Säure ausgewählt sind.

15. Zusammensetzung nach Anspruch 7, **dadurch gekennzeichnet, dass** die heterocyclischen Oxidationsbasen unter den Pyridinderivaten, Pyrimidinderivaten, Pyrazolderivaten, Pyrazolo-pyrimidinderivaten und deren Additionssalzen mit einer Säure ausgewählt sind.

16. Zusammensetzung nach einem der vorhergehenden Ansprüche, **dadurch gekennzeichnet, dass** die Oxidationsbase(n) 0,0005 bis 12 Gew.-% des Gesamtgewichts der Farbmittelzusammensetzung ausmachen.

17. Zusammensetzung nach Anspruch 16, **dadurch gekennzeichnet, dass** die Oxidationsbase(n) 0,005 bis 6 Gew.-% des Gesamtgewichts der Farbmittelzusammensetzung ausmachen.

18. Zusammensetzung nach einem der vorhergehenden Ansprüche, **dadurch gekennzeichnet, dass** das 2-Amino-4-N-(β-hydroxyethyl)-amino-anisol und/ oder sein(e) Additionssalz(e) mit einer Säure 0,0001 bis 8 Gew.-%, bezogen auf das Gesamtgewicht der Zusammensetzung, ausmachen.

19. Zusammensetzung nach Anspruch 18, **dadurch gekennzeichnet, dass** das 2-Amino-4-N-(β-hydroxyethyl)amino-anisol und/oder sein(e) Additionssalz(e) mit einer Säure in Konzentrationen von 0,005 bis 5 Gew.-%, bezogen auf das Gesamtgewicht der Zusammensetzung, vorliegen.

20. Zusammensetzung nach einem der vorhergehenden Ansprüche, **dadurch gekennzeichnet, dass** sie einen oder mehrere weitere Kuppler, die von dem 2-Amino-4-N-(β-hydroxyethyl)amino-anisol verschieden sind, und/oder Direktfarbstoffe enthält.

21. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

22. Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

23. Zusammensetzung nach einem der vorhergehenden Ansprüche, **dadurch gekennzeichnet, dass** sie einen pH-Wert im Bereich von 4 bis 11 aufweist.

24. Verfahren zum Färben von Keratinfasern und insbesondere menschlichen Keratinfasern, wie dem Haar, **dadurch gekennzeichnet, dass** auf die Fasern mindestens eine gebrauchsfertige Farbmittelzusammensetzung nach einem der vorhergehenden Ansprüche während einer Zeitspanne, die zur Entwicklung der gewünschten Färbung ausreichend ist, aufgebracht wird.

25. Verfahren nach Anspruch 24, **dadurch gekennzeichnet, dass** es einen vorbereitenden Schritt umfasst, der darin besteht, einerseits eine Zusammensetzung (A), die in einem zum Färben geeigneten Medium mindestens eine Oxidationsbase und das 2-Amino-4-N-(β-hydroxyethyl)amino-anisol und/ oder mindestens eines seiner Additionssalze mit einer Säure enthält, und andererseits eine Zusammensetzung (B) getrennt voneinander aufzubewahren, die in einem zum Färben geeigneten Medium mindestens ein Enzym vom Laccase-Typ enthält, und diese bei der Anwendung zu vermischen, bevor das Gemisch auf die Keratinfasern aufgebracht wird.

26. Vorrichtung mit mehreren Abteilungen oder "Kit" zum Färben, **dadurch gekennzeichnet, dass** sie eine erste Abteilung mit der in Anspruch 25 definierten Zusammensetzung (A) und eine zweite Abteilung mit der in Anspruch 25 definierten oxidierenden Zusammensetzung (B) enthält.

## Claims

1. Ready-to-use composition for the oxidation dyeing of keratinous fibres, and in particular human keratinous fibres such as hair, **characterized in that** it comprises, in a medium appropriate for dyeing:
- at least one oxidation base,
- 2-amino-4-N-(β-hydroxyethyl)aminoanisole and/or at least one of its addition salts with an acid as coupler,
- at least one enzyme of the laccase type.

2. Composition according to Claim 1, **characterized in that** the laccase is chosen from laccases of plant origin, animal origin, fungal origin or bacterial origin and from the laccases obtained by biotechnology.

3. Composition according to either of Claims 1 and 2, **characterized in that** the laccase is of plant origin and chosen from the laccases present in the extracts of Anacardiaceae, Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Indian pipe), Aesculus sp., Acer pseudoplatanus, Prunus persica and Pistacia palaestina.

4. Composition according to Claim 1 or 2, **characterized in that** the laccase is of fungal origin or obtained by biotechnology.

5. Composition according to Claim 4, **characterized in that** the laccase is chosen from the laccases derived from Polyporus versicolor, Rhizoctonia praticola, Rhus vernicifera, Scytalidium, Polyporus pinsitus, Myceliophtora thermophila, Rhizoctonia solani, Pyricularia orizae, Trametes versicolor, Fomes fomentarius, Chaetomium thermophile, Neurospora crassa, Colorius versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitius squalens, and from variants thereof.

6. Composition according to any one of the preceding claims, **characterized in that** the quantity of laccase(s) is between 0.5 and 200 Lacu per 100 g of dyeing composition.

7. Composition according to any one of the preceding claims, **characterized in that** the oxidation base(s) are chosen from para-phenylenediamines, double bases, para-aminophenols, ortho-aminophenols and heterocyclic oxidation bases.

8. Composition according to Claim 7, **characterized in that** the para-phenylenediamines are chosen from the compounds of formula (I), and their addition salts with an acid: 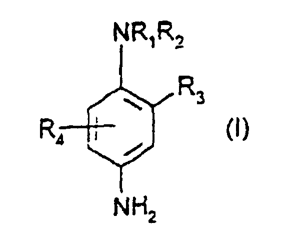 in which:
- R₁ represents a hydrogen atom, a C₁-C₄ alkyl radical, a monohydroxy(C₁-C₄ alkyl) radical, a polyhydroxy(C₂-C₄ alkyl) radical, a (C₁-C₄)alkoxy(C₁-C₄)alkyl radical, a C₁-C₄ alkyl radical substituted with a nitrogen-containing group, a phenyl radical or a 4'-aminophenyl radical;
- R₂ represents a hydrogen atom, a C₁-C₄ alkyl radical, a monohydroxy(C₁-C₄ alkyl) radical, a polyhydroxy(C₂-C₄ alkyl) radical, a (C₁-C₄)alkoxy(C₁-C₄)alkyl radical or a C₁-C₄ alkyl radical substituted with a nitrogen-containing group;
- R₃ represents a hydrogen atom, a halogen atom such as a chlorine, bromine, iodine or fluorine atom, a C₁-C₄ alkyl radical, a monohydroxy(C₁-C₄ alkyl) radical, a hydroxy(C₁-C₄ alkoxy) radical, an acetylamino (C₁-C₄ alkoxy) radical, a mesylamino(C₁-C₄ alkoxy) radical or a carbamoylamino (C₁-C₄ alkoxy) radical,
- R₄ represents a hydrogen or halogen atom or a C₁-C₄ alkyl radical.

9. Composition according to Claim 8, **characterized in that** the para-phenylenediamines of formula (I) are chosen from para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine, N-(β-methoxyethyl)-para-phenylenediamine, and their addition salts with an acid.

10. Composition according to Claim 7, **characterized in that** the double bases are chosen from the compounds of formula (II), and their addition salts with an acid: 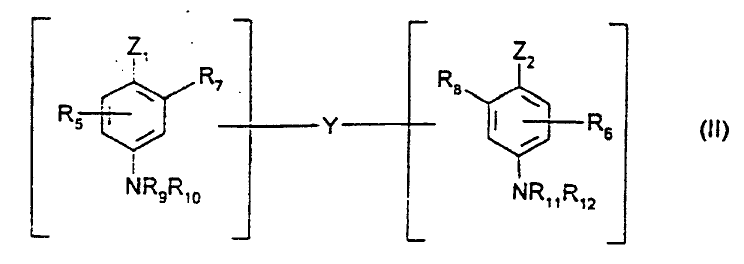 in which:
- Z₁ and Z₂, which are identical or different, represent a hydroxyl or -NH₂ radical which may be substituted with a C₁-C₄ alkyl radical or with a linking arm Y;
- the linking arm Y represents a linear or branched alkylene chain comprising from 1 to 14 carbon atoms, which may be interrupted by or which may end with one or more nitrogen-containing groups and/or one or more heteroatoms such as oxygen, sulphur or nitrogen atoms, and optionally substituted with one or more hydroxyl or C₁-C₆ alkoxy radicals;
- R₅ and R₆ represent a hydrogen or halogen atom, a C₁-C₄ alkyl radical, a monohydroxy (C₁-C₄ alkyl) radical, a polyhydroxy(C₂-C₄ alkyl) radical, an amino(C₁-C₄ alkyl) radical or a linking arm Y;
- R₇, R₈, R₉, R₁₀, R₁₁ and R₁₂, which are identical or different, represent a hydrogen atom, a linking arm Y or a C₁-C₄ alkyl radical;
it being understood that the compounds of formula (II) contain only one linking arm Y per molecule.

11. Composition according to Claim 10, **characterized in that** the double bases of formula (II) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine, 1,8-bis(2,5-diaminophenoxy)-3,5-dioxaoctane, and their addition salts with an acid.

12. Composition according to Claim 7, **characterized in that** the para-aminophenols are chosen from the compounds of formula (III), and their addition salts with an acid: 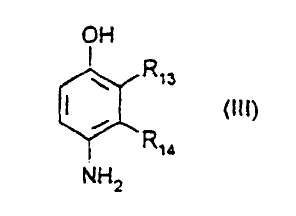 in which:
- R₁₃ represents a hydrogen or halogen atom, a C₁-C₄ alkyl, monohydroxy(C₁-C₄ alkyl), (C₁-C₄) alkoxy (C₁-C₄) alkyl, amino (C₁-C₄ alkyl) or hydroxy (C₁-C₄) alkylamino (C₁-C₄ alkyl) radical,
- R₁₄ represents a hydrogen or halogen atom, a C₁-C₄ alkyl, monohydroxy(C₁-C₄ alkyl), polyhydroxy (C₂-C₄ alkyl), amino(C₁-C₄ alkyl), cyano(C₁-C₄ alkyl) or (C₁-C₄)alkoxy(C₁-C₄) alkyl radical, it being understood that at least one of the radicals R₁₃ or R₁₄ represents a hydrogen atom.

13. Composition according to Claim 12, **characterized in that** the para-aminophenols of formula (III) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol, 4-amino-2-fluoro-phenol, and their addition salts with an acid.

14. Composition according to Claim 7, **characterized in that** the ortho-aminophenols are chosen from 2-aminophenol, 2-amino-5-methylphenol, 2-amino-6-methylphenol, 5-acetamido-2-aminophenol, and their addition salts with an acid.

15. Composition according to Claim 7, **characterized in that** the heterocyclic bases are chosen from pyridine derivatives, pyrimidine derivatives, pyrazole derivatives, pyrazolopyrimidine derivatives, and their addition salts with an acid.

16. Composition according to any one of the preceding claims, **characterized in that** the oxidation base(s) represent from 0.0005 to 12% by weight of the total weight of the dyeing composition.

17. Composition according to Claim 16, **characterized in that** the oxidation base(s) represent from 0.005 to 6% by weight of the total weight of the dyeing composition.

18. Composition according to any one of the preceding claims, **characterized in that** the 2-amino-4-N-(β-hydroxyethyl)aminoanisole and/or the or its addition salts with an acid represent from 0.0001 to 8% by weight of the total weight of the dyeing composition.

19. Composition according to Claim 18, **characterized in that** the 2-amino-4-N-(β-hydroxyethyl)aminoanisole and/or the or its addition salts with an acid represent from 0.005 to 5% by weight of the total weight of the dyeing composition.

20. Composition according to any one of the preceding claims, **characterized in that** it contains one or more other couplers different from 2-amino-4-N-(β-hydroxyethyl)aminoanisole and/or direct dyes.

21. Composition according to any one of the preceding claims, **characterized in that** the addition salts with an acid are chosen from hydrochlorides, hydrobromides, sulphates and tartrates, lactates and acetates.

22. Composition according to any one of the preceding claims, **characterized in that** the medium appropriate for dyeing consists of water or of a mixture of water and at least one organic solvent.

23. Composition according to any one of the preceding claims, **characterized in that** it has a pH of between 4 and 11.

24. Method of dyeing keratinous fibres, and. in particular human keratinous fibres such as hair, **characterized in that** at least one ready-to-use dyeing composition as defined in any one of the preceding claims is applied to the said fibres for a sufficient time to develop the desired colour.

25. Method according to Claim 24, **characterized in that** it comprises a preliminary step consisting in storing in a separate form, on the one hand, a composition (A) comprising, in a medium appropriate for dyeing, at least one oxidation base and 2-amino-4-N-(β-hydroxyethyl)aminoanisole and/or at least one of its addition salts with an acid and, on the other hand, a composition (B) containing, in a medium appropriate for dyeing, at least one enzyme of laccase type, and then in mixing them at the time of use before applying this mixture to the keratinous fibres.

26. Multicompartment device or dyeing "kit", **characterized in that** it comprises a first compartment containing the composition (A) as defined in Claim 25 and a second compartment containing the composition (B) as defined in Claim 25.
